# EUROPÄISCHE PATENTANMELDUNG

(11) **EP 4 760 763 A1**
(43) Veröffentlichungstag der Anmeldung: **17.06.2026**
(21) Anmeldenummer: 24218772.2
(22) Anmeldetag: 10.12.2024
(51) Int. Cl.: H01H 9/02, A61M 5/172, G16H 20/17, A61M 5/142, H01H 13/02, A61M 5/14

(54) **HANDGERÄT MIT AKTIVIERUNGSSCHALTER ZUR PATIENTENKONTROLLIERTEN ANALGESIE**

(71) Anmelder: B. Braun Melsungen AG, 34212 Melsungen (DE)
(72) Erfinder: SCHWARZ, Jan, 34212 Melsungen (DE); BUERGER, Maria, 34323 Malsfeld (DE); BARKOWSKI, Peter, 34212 Melsungen (DE)
(74) Vertreter: Winter, Brandl - Partnerschaft mbB

(57) **Zusammenfassung**

Die Offenbarung betrifft ein Handgerät (1) zur patientenkontrollierten Analgesie mit einem Griff (4), welcher vorgesehen und ausgebildet ist, von einem Patienten mit einer Hand gegriffen und gehalten zu werden, und welcher ein Volumen mit einem Volumenschwerpunkt (19) einschließt oder definiert, wobei der Griff (4) einen ersten Griffabschnitt (4a) aufweist, welcher vorgesehen und ausgebildet ist, von einem Daumen und einem Zeigefinger oder einem Mittelfinger des Patienten derart gegriffen zu werden, dass ein an einer ersten Großfläche (2a) des Griffs (4) vorgesehener Aktivierungsschalter (8) von dem Daumen des Patienten betätigbar ist, während der Zeigefinger oder der Mittelfinger des Patienten in Anlage mit einer an einer zweiten Großfläche (3a) des Griffs (4) vorgesehenen definierten Fingeraufnahme (16) bringbar ist, und wobei der Griff (4) einen Massenschwerpunkt (23) aufweist, welcher bezüglich des Volumenschwerpunkts (19) des Griffs (4) in einer Richtung weg von dem ersten Griffabschnitt (4a) verschoben ist und sich in einem zweiten Griffabschnitt (4b) des Griffs (4) befindet.

## Beschreibung

### Technisches Gebiet

Die vorliegende Offenbarung betrifft ein Handgerät (Bedienteil) mit einem Aktivierungsschalter (Schaltknopf) zur patientenkontrollierten Analgesie (PCA).

### Hintergrund der Offenbarung

Die patientenkontrollierte Analgesie, kurz PCA, ist eine vom Patienten selbst gesteuerte, parenterale oder rückenmarksnahe Zufuhr von Analgetika mit Hilfe einer elektronisch gesteuerten Infusionspumpe. Auf Knopfdruck kann der Patient eine vorprogrammierte Schmerzmitteldosis abrufen und sich selbst zuführen.

Meistens erfolgt die Abgabe der Schmerzmittel (in der Regel hochpotente Analgetika aus der Gruppe der Opiate) über einen intravenösen Zugang, an den die Infusionspumpe angeschlossen ist. Der Katheter wird direkt über eine Y-Stück an den bestehenden zentralen Venenkatheter (ZVK) oder die periphere Venenverweilkanüle angeschlossen.

Der Patient kann bei dieser Schmerztherapie per Knopfdruck einen sogenannten Bolus applizieren. Der behandelnde Arzt legt dabei fest, wie hoch die applizierte Dosis ist. Durch ein Sperrintervall wird verhindert, dass ein weiterer Bolus in kurzer Folge injiziert werden kann. Auf diese Weise wird eine versehentliche oder vorsätzliche Überdosierung verhindert. Der Patient kann seine Schmerzmedikation dadurch bedarfsgerecht verabreichen und z.B. auch pausieren, wenn er keine oder nur geringe Schmerzen hat.

Diese Form der Schmerztherapie hat sich seit den 80er Jahren als weltweiter Standard in der postoperativen Phase durchgesetzt. Außerdem kommt sie häufig beim chronischen Schmerzsyndrom und im Rahmen der Palliativmedizin zum Einsatz.

### Stand der Technik

Aus dem Stand der Technik sind verschieden gestaltete Handgeräte (Bedienteile) bekannt, die der Patient in der Hand hält, um dann den daran angeordneten Bedienschalter zu drücken.

Die WO 2021/064415 A1 offenbart ein Handgerät mit einem Griff zum Greifen durch eine Hand des Patienten und mit einem Schalter zur Bedienung durch den Daumen des Patienten. Der Griff hat Mulden für einzelne Finger. An einem vom Signalleitungsanschluss entfernten (oberen) Endabschnitt ist der Schalter in geneigter Stellung angeordnet. Der Schalter ist im Griff versenkt.

Die US 2013/0123745 A1 offenbart ein Handgerät mit einem Griff und mit einem Schaltknopf. Der Griff hat eine Mulde für einen Finger. An einem vom Signalleitungsanschluss entfernten (oberen) Endabschnitt ist der Schaltknopf in geneigter Stellung angeordnet. Der Schaltknopf ist hervorstehend bzw. erhaben gegenüber dem Griff. Es wird auch eine als Findelicht dienende Beleuchtung vorgeschlagen.

Aus hausinternem Stand der Technik ist ein Handgerät mit einem im weitesten Sinne kastenförmigen Griff und mit einem Schaltknopf bekannt. Genauer gesagt hat der Griff zwei einander gegenüberliegende etwa parallele Großflächen. An dem unteren Bereich des so gebildeten Griffs ist der Signalleitungsanschluss angeordnet. An einer der beiden Großflächen ist der Schaltknopf in die betroffene Großfläche integriert.

Es hat sich an derartigen Handgeräten des Standes der Technik jedoch gezeigt, dass die Ergonomie und das Handling für den Patienten suboptimal sind.

### Kurzbeschreibung der Offenbarung

Aufgabe der vorliegenden Offenbarung ist es, ein in Ergonomie und Handling verbessertes Handgerät bereitzustellen.

Diese Aufgabe wird mit der Merkmalskombination des Anspruchs 1 gelöst.

Das Handgerät (Bedienteil, PCA-Button) gemäß der Offenbarung ist zur patientenkontrollierten Analgesie ausgelegt und eingerichtet. Es hat einen Griff, welcher vorgesehen und ausgebildet ist, von einem Patienten mit einer Hand gegriffen und gehalten zu werden, und welcher ein Volumen mit einem Volumenschwerpunkt einschließt oder definiert. Der Griff weist einen ersten (oberen) Griffabschnitt auf, welcher vorgesehen und ausgebildet ist, einerseits von einem Daumen und andererseits von einem Zeigefinger oder einem Mittelfinger des Patienten derart gegriffen zu werden, dass ein an einer ersten Großfläche des Griffs vorgesehener Aktivierungsschalter von dem Daumen des Patienten betätigbar ist, während der Zeigefinger oder der Mittelfinger des Patienten in Anlage mit einer an einer zweiten Großfläche des Griffs vorgesehenen definierten Fingeraufnahme bringbar ist. Entsprechend ist der an der ersten Großfläche des Griffs vorgesehene Aktivierungsschalter (Taster) von dem Daumen des Patienten betätigbar. Der Zeigefinger oder der Mittelfinger des Patienten ist in Anlage mit der an der zweiten Großfläche des Griffs vorgesehenen definierten Fingeraufnahme bringbar. Die beiden Großflächen sind vorzugsweise einander gegenüberliegend. Der Griff weist einen Massenschwerpunkt auf, welcher bezüglich des Volumenschwerpunkts des Griffs in einer Richtung weg von dem ersten Griffabschnitt verschoben ist und sich in einem zweiten (unteren) Griffabschnitt des Griffs befindet. Damit ist der Massenschwerpunkt des Handgerätes gegenüber denjenigen des Standes der Technik in Richtung zur Handunterkante des Patienten verschoben, wodurch das Handgerät in die Hand des Patienten hineinfällt.

Bevorzugt weist der Griff des Handgeräts den ersten Griffabschnitt zur Anlage zumindest des Daumens und des Zeigefingers oder Mittelfingers und den zweiten Griffabschnitt zur Anlage zumindest des Ringfingers auf.

Der erste Griffabschnitt kann eine größere Querschnittsfläche als der zweite Griffabschnitt haben. Damit ist der erste Griffabschnitt bevorzugt dicker und/oder größer, insbesondere breiter als der zweite Griffabschnitt. Der Querschnitt der beiden Griffabschnitte ist bevorzugt nicht konstant und gleichbleibend, insbesondere ist der Querschnitt des Griffs bei einer Betrachtung entlang seiner Längsachse bevorzugt vom zweiten Griffabschnitt zum ersten Griffabschnitt stetig zunehmend. Der Griff hat die erste Großfläche und die dieser gegenüberliegende zweite Großfläche, die sich bevorzugt beide vom ersten Griffabschnitt bis zum zweiten Griffabschnitt erstecken, bzw. in anderen Worten jeweils Bestandteil bzw. Abschnitt sowohl des ersten Griffabschnitts als auch des zweiten Griffabschnitts sind. In der ersten Großfläche ist am ersten Griffabschnitt der Aktivierungsschalter angeordnet. Offenbarungsgemäß ist ein Massenschwerpunkt des Griffs einschließlich dem Aktivierungsschalter, welcher Bestandteil des Griffs ist, aber ohne Berücksichtigung einer eventuellen Signalleitung, welche nicht Bestandteil des Griffs ist, im zweiten Griffabschnitt des Griffs angeordnet.

Der Begriff "Großfläche" ist vorzugsweise derart zu verstehen, dass deren Längen- und Breitenerstreckung (wesentlich) größer als eine Tiefe des Griffs ist.

Vorzugsweise sind die beiden Großflächen trapezförmig oder dreieckförmig ausgestaltet bzw. weisen eine Trapez- oder Dreieckform auf, derart, sich der Griff von dem ersten Griffabschnitt hin zu dem zweiten Griffabschnitt verjüngt. Eine derartige trapezförmige oder dreieckförmige Ausbildung des Griffs hat sich als in großem Maße ergonomisch für einen Patienten herausgestellt.

Wenn in dem zweiten Griffabschnitt ein Gewichtskörper angeordnet ist, lässt sich der Massenschwerpunkt des Handgerätes besonders weit in Richtung zur Handunterkante des Patienten verschieben, wodurch das Handgerät besonders schnell und definiert in die Hand des Patienten hineinfällt.

Fertigungstechnisch bevorzugt ist es, wenn der Gewichtskörper von einer Vergussmasse, beispielsweise aus Harz, bevorzugt Epoxidharz, gebildet ist, die durch eine im Griff angeordnete Öffnung in den zweiten Griffabschnitt eingespritzt oder eingegossen ist.

Der Griff kann als Gehäuse ausgebildet sein. Wenn das Gehäuse allseitig geschlossen (z.B. wasserfest) ist, ist eine Entlüftungsöffnung im Griff besonders bevorzugt, damit Luft aus dem Gehäuse entweichen kann, wenn die Vergussmasse eingespritzt oder eingegossen wird.

In den meisten Fällen ist eine Signalleitung an den zweiten Griffabschnitt des Griffs angeschlossen, die den Griff mit einer Infusionspumpe verbindet. Vorzugsweise ist die Signalleitung mit mindesten einem Strang aus Para-Aramid (Kevlar) verstärkt, der im Innern des Griffs an diesem befestigt ist. Damit ist eine besonders hohe Abreißfestigkeit des Griffs gegenüber der Signalleitung gegeben.

Insbesondere wenn die Signalleitung eine gewisse Elastizität bzw. Steifigkeit hat, ist es besonders bevorzugt, wenn die Signalleitung an den zweiten Griffabschnitt angeschlossen ist. Dann ist die offenbarungsgemäße Verlagerung des Massenschwerpunkt in den zweiten Griffabschnitt vorteilhaft, da dieser dann auch gegen eine elastische Kraft der Signalleitung in die Hand gedrückt wird bzw. fällt, und so in Anlage mit der Handfläche kommt und leicht bzw. einfach gegriffen werden kann.

Wenn die beiden Großflächen des Griffs etwa parallel zueinander sind, ist das ganzheitliche Optimum der Ergonomie des Handgerätes einerseits für Linkshänder und andererseits für Rechtshänder gegeben.

Vorzugsweise hat jede der beiden Großflächen drei oder vier Ränder. Zwei Ränder erstrecken sich jeweils vom ersten zum zweiten Griffabschnitt, wobei die beiden Ränder jeder Großfläche im ersten, vorzugsweise breiteren, Griffabschnitt einen größeren Abstand zueinander haben, als im zweiten, vorzugsweise schmaleren, Griffabschnitt.

Wenn der Griff als Gehäuse weitergebildet ist, ist es besonders bevorzugt, wenn die beiden Großflächen an jeweiligen Halbschalen des Gehäuses gebildet oder befestigt sind, die jeweils einen umlaufenden Schalenrand haben. Die Halbschalen können gleich groß sein.

Die definierte Fingeraufnahme ist besonders bevorzugt als in dem Griff/ dem ersten Griffabschnitt ausgebildete, insbesondere als in der zweiten Halbschale des Griffs ausgebildete, rinnenförmige oder trogförmige Vertiefung gebildet, die sich durchgehend zwischen den beiden Rändern der zweiten Großfläche erstreckt. Damit kann das Handgerät am Zeigefinger oder Mittelfinger des Patienten entlang der Länge des Griffs eine klar definierte Anlageposition (Anlagehöhe) finden, unabhängig davon, in wieweit der genannte Finger länger als die Vertiefung ist.

Vorzugsweise weist die Vertiefung ausgehend von einer Stelle mit minimaler Größe in Richtung zu den beiden Rändern jeweils zunehmende Größe auf. "Größe" der Vertiefung bedeutet Tiefe und/oder Breite. Damit kann das Handgerät zwischen zwei abgewinkelten Gliedern des Zeigefingers eine besonders klar definierte Anlageposition finden.

Wenn die Stelle mit minimaler Größe mittig zwischen den beiden Rändern der zweiten Großfläche angeordnet ist, dann ist das ganzheitliche Optimum der Ergonomie des Handgerätes einerseits für Linkshänder und andererseits für Rechtshänder gegeben.

Besonders bevorzugt ist die Stelle mit minimaler Größe gegenüber dem Aktivierungsschalter angeordnet. Dadurch ist im Zusammenwirken der Abwinkelung im Zeigefinger mit dem Daumen quasi ein Schwenklager für das Handgerät gebildet, dessen Schwenkachse sich durch die Stelle mit minimaler Größe und den Aktivierungsschalter erstreckt. So kann der offenbarungsgemäß von dieser Stelle beabstandete Massenschwerpunkt ein definiertes Schwenken des Griffs bewirken. Damit kann der Griff, insbesondere der zweite Griffabschnitt besonders angenehm und ergonomisch in die Handfläche des Patienten gelangen bzw. fallen.

Wenn der Aktivierungsschalter mittig zwischen den beiden Rändern der ersten Großfläche angeordnet ist, dann ist das ganzheitliche Optimum der Ergonomie des Handgerätes einerseits für Linkshänder und andererseits für Rechtshänder gegeben.

Wenn der Aktivierungsschalter ein Folienschalter ist, kann das Handgerät wasserfest ausgebildet sein. Insbesondere wird der Griff dazu mit den Halbschalen und den Halbschalenrändern ausgebildet, die dann dichtend aneinander anliegen. Somit kann das Gehäuse allseitig geschlossen sein.

Die zweite Großfläche kann einstückig an der zweiten Halbschale gebildet sein, die erste Großfläche kann ein extra Bauteil (dekorative Folie) sein, das dichtend an der ersten Halbschale befestigt ist.

Vorzugsweise ist der Aktivierungsschalter gegenüber der ersten Großfläche erhaben und nach außen hervorstehend. Damit ist die Auffindbarkeit des Aktivierungsschalters für den Patienten bei Dunkelheit und/oder ohne Hinsehen verbessert.

Vorzugsweise weist der Aktivierungsschalter eine Signalfarbe auf, die von einer Farbe der ersten Großfläche (kontrastreich) abweicht. Z.B. kann ein orangener Aktivierungsschalter auf weißer erster Großfläche vorgesehen sein. Damit ist die Auffindbarkeit des Aktivierungsschalters für den Patienten verbessert.

Vorzugsweise ist an der ersten Großfläche oder an dem Aktivierungsschalter eine als Findelicht ausgebildete z.B. weiße (LED-) Beleuchtung vorgesehen. Damit ist die Auffindbarkeit des Aktivierungsschalters für den Patienten bei Dunkelheit verbessert.

Bei einer bevorzugten Ausgestaltung ist an der ersten Großfläche oder an dem Aktivierungsschalter eine als Freigabelicht ausgebildete, z.B. grüne, (LED) Beleuchtung vorgesehen. Diese signalisiert, wenn bzw. dass genug Zeit verstrichen ist, so dass der Patient den nächsten Bolus verabreichen kann.

Vorzugsweise ist dieses Freigabelicht optional zuschaltbar.

Bei einer bevorzugten Ausgestaltung hat der Griff eine Höhe von 90 bis 110 mm. Damit erstreckt sich der Griff, sofern der Zeigefinger in der Vertiefung ist, an der Seite des kleinen Fingers nicht aus einer durchschnittlichen Hand heraus, so dass der kleine Finger als Anlage für die zweite Stirnseite (am zweiten Griffabschnitt) dienen kann.

### Kurzbeschreibung der Figuren

Figur 1 zeigt ein Handgerät gemäß dem Ausführungsbeispiel der vorliegenden Offenbarung mit einer Hand in einer Ansicht;
Figur 2 zeigt das Handgerät aus Figur 1 in einer weiteren Ansicht;
Figur 2a zeigt einen Querschnitt des Handgerätes aus Figur 2;
Figur 3 zeigt das Handgerät aus den vorhergehenden Figuren in einer weiteren Ansicht; und
Figur 4 zeigt das Handgerät ohne eine erste Großfläche in einer Ansicht gemäß Figur 1.

### Beschreibung des Ausführungsbeispiels

Nachstehend wird ein Ausführungsbeispiel der vorliegenden Offenbarung auf der Basis der zugehörigen Figuren beschrieben.

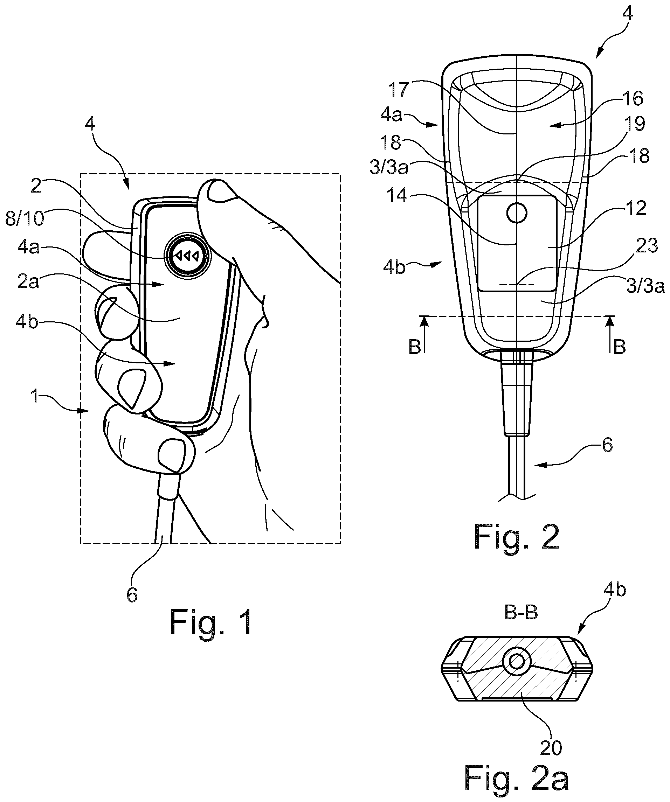

Figur 1 zeigt ein Handgerät 1 gemäß dem Ausführungsbeispiel der vorliegenden Offenbarung mit einer Hand eines Patienten. Gezeigt ist eine Ansicht auf eine erste Großfläche 2a, die an einer ersten Halbschale 2 eines gehäuseartigen Griffs 4 befestigt ist. Der Griff 4 hat eine (in Figur 1 senkrecht angeordnete) Länge von nur etwa 90-110 mm, sodass er rückseitig vorzugsweise nur von dem Zeigefinger und dem Mittelfinger und dem Ringfinger des Patienten umgriffen wird. Der Griff 4 erstreckt sich also nicht bis zum kleinen Finger des Patienten, sodass der kleine Finger den Griff 4 vorzugsweise in Schwerkraftrichtung nach unten abstützt und dabei einen Anschluss einer Signalleitung 6 umgreift. Über die Signalleitung 6 ist der Griff 4 mit einer nicht gezeigten Infusionspumpe für Schmerzmittel elektrisch verbunden.

Der Griff 4 lässt sich in einen in Figur 1 oberen ersten Griffabschnitt 4a und einen in Figur 1 unteren zweiten Griffabschnitt 4b unterteilen. Der erste Griffabschnitt 4a ist breiter als der zweite Griffabschnitt 4b. Am zweiten Griffabschnitt 4b, genauer gesagt an einer zweiten Stirnseite des Griffs 4b (in Figur 1 unten) ist der Anschluss für die Signalleitung 6 gebildet, der von dem kleinen Finger umgriffen werden kann.

Die erste Halbschale 2 des gehäuseartigen Griffs 4 ist von der im Wesentlichen ebenen erste Großfläche 2a abgedeckt, die vorliegend als Extrabauteil ausgebildet und an der ersten Halbschale 2 befestigt ist. Die erste Großfläche 2a kann alternativ jedoch auch ein (integraler) Abschnitt bzw. Bestandteil der ersten Halbschale 2 sein. In die erste Großfläche 2a ist ein als Folienschalter ausgebildeter Aktivierungsschalter 8 integriert. Ein Drücken des Aktivierungsschalters 8 durch den Daumen des Patienten bewirkt die Gabe eines Bolus eines Schmerzmittels über die an die Signalleitung 6 angeschlossene (nicht gezeigte) Infusionspumpe.

Für die Erkennbarkeit bei Tageslicht oder bei Raumbeleuchtung ist der Aktivierungsschalter 8 mit einer Signalfarbe versehen, die sich kontrastreich von der übrigen ersten Großfläche 2a absetzt. Beim gezeigten Ausführungsbeispiel ist der Aktivierungsschalter 8 orange gefärbt, während die übrige erste Großfläche 2a weiß ist.

Für die Erkennbarkeit bei Dunkelheit hat der Aktivierungsschalter 8 eine als Findelicht 10 dienende LED Beleuchtung.

Zur Anzeige einer Freigabe, dass ein nächster Bolus verabreicht werden kann, ist eine als Freigabelicht dienende LED-Beleuchtung in der ersten Großfläche 2a vorgesehen. Beim gezeigten Ausführungsbeispiel ist das Freigabelicht grün und optional zuschaltbar.

Figur 2 zeigt das Handgerät 1 aus Figur 1 in einer weiteren Ansicht auf eine rückseitige zweite im wesentlichen ebene Großfläche 3a mit einem Etikett 12. im Bereich des ersten Griffabschnitts 4a ist an der zweiten Großfläche 3a in Querrichtung zu einer Längsachse 14 eine Einkerbung oder Vertiefung 16 vorgesehen, die zur Auflage auf einen Finger ausgelegt ist. Diese Vertiefung 16 besitzt eine zur Längsachse 14 spiegelsymmetrische Doppelkegel-Struktur (Hyperboloid), die sich auf einen Finger des Anwenders, im Normalfall den Zeigefinger, ausrichtet. Genauer gesagt hat die Vertiefung 16 eine mittlere Stelle 17 mit minimaler Größe, an der die (in Figur 2 ersichtliche) Breite und darüber hinausgehend auch die Tiefe der Vertiefung 16 minimal ist. Ausgehend von dort erstrecken sich die beiden Kegel der Vertiefung 16 mit zunehmender Breite und Tiefe bis zum jeweiligen Rand 18 der Großfläche 3a. Diese Ausrichtungsgeometrie im Zusammenhang mit einem in den zweiten Griffabschnitt 4b verschobenen Massenschwerpunkt 23 bewirkt, dass sich der Griff 4 in der Hand ausrichtet und sich der Aktivierungsschalter 8 mittels des Daumens drücken lässt.

Der Griff 4 einschließlich dem Aktivierungsschalter 8 bildet ein (volumenbehaftetes) Gehäuse, das einen Volumenschwerpunkt 19 hat. Da der erste vom Anschluss der Signalleitung 6 entfernte Abschnitt 4a des Griffs 4 breiter als der zweite Abschnitt 4b ist, liegt der Volumenschwerpunkt 19 von dem Anschluss der Signalleitung 6 bzw. dem unteren Rand weiter entfernt als von dem oberen Rand. Beim gezeigten Ausführungsbeispiel definiert der Volumenschwerpunkt 19 eine Trennebene senkrecht zur Längsachse 14, die die Grenze zwischen den beiden Abschnitte 4a, 4b bildet. Die Trennebene kann auch senkrecht zu den beiden Großflächen 2a, 3a und /oder im Fall des gezeigten etwa trapezförmigen Griffs 4 parallel zum oberen und unteren (kurzen) Rand angeordnet sein. Der Griff 4 weist entsprechend den Massenschwerpunkt 23 auf, welcher bezüglich des Volumenschwerpunkts 19 des Griffs 4 in einer Richtung weg von dem ersten Griffabschnitt 4a verschoben ist und sich in einem zweiten Griffabschnitt 4b des Griffs 4 befindet.

Figur 2a zeigt einen Querschnitt des zweiten Griffabschnitts 4b gemäß der Linie B-B aus Figur 2. Genauer gesagt verläuft die Schnittebene B-B durch einen eingegossenen Gewichtskörper 20, der die offenbarungsgemäße Verschiebung des Massenschwerpunkts 23 entlang der Längsachse 14 in Richtung zur Signalleitung 6 bewirkt.

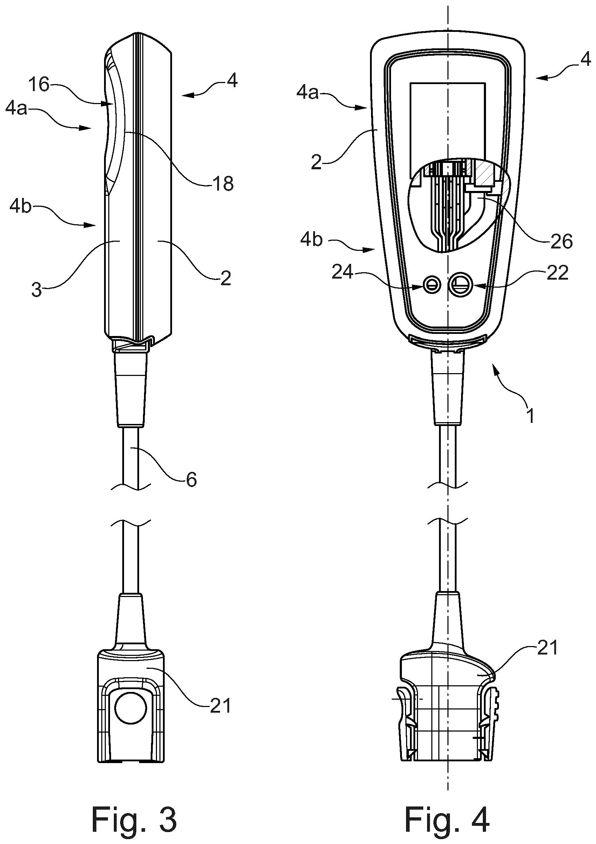

Figur 3 zeigt das Handgerät 1 aus den vorhergehenden Figuren in einer seitlichen Ansicht. Dabei ist zu erkennen, dass die beiden Großflächen 2a, 3a zueinander parallel sind, sodass ein etwa kasten- oder schachtelförmiges Gehäuse entsteht, aus dem der Griff 4 gebildet ist. Die beiden Halbschalen 2, 3 haben jeweils einen umlaufenden Schalenrand, mit dem die beiden Halbschalen 2, 3 vollumfänglich dichtend aneinander anliegen. In Figur 3 ist auch die Vertiefung 16 und deren verrundeter Übergang zum gezeigten Rand 18 zu erkennen.

Die Signalleitung 6 ist über eines Steckkontakt 21 mit der (nicht gezeigten) Infusionspumpe elektrisch verbindbar.

Figur 4 zeigt das Handgerät 1 in einer Ansicht auf die erste Halbschale 2 des Griffs 4, an der die erste im Wesentlichen ebene (in Figur 1 gezeigte) Großfläche 2a noch nicht befestigt ist. Dabei ist zu erkennen, dass in der ersten Halbschale 2 im Bereich des zweiten Griffabschnitts 4b einerseits eine Öffnung 22 gebildet ist, durch die die Vergussmasse zur Bildung des Gewichtskörpers 20 ins Innere des zweiten Griffabschnitts 4b eindringen kann. Weiterhin ist eine Entlüftungsöffnung 24 gezeigt, über die die verdrängte Luft des wasserfesten Griffs 4 entweichen kann.

Die Signalleitung 6 ist mit mindestens einem Band oder Strang 26 aus Para-Aramid (Kevlar) verstärkt, der im Innern des Griffs 4 an dessen Halbschalen 2, 3 befestigt ist. Damit ist eine besonders hohe Abreißfestigkeit des Griffs 4 gegenüber der Signalleitung gegeben. Aufgrund eines Ausbruchs in der ersten Gehäuseschale 2 ist ein kurzer Abschnitt dieses Strangs 26 ersichtlich.

Offenbart ist ein Handgerät 1 mit einem Aktivierungsschalter 8, der auch als Taster bezeichnet werden kann. Das Handgerät 1 hat einen gehäuseartigen Griff 4 mit zwei zueinander parallelen Großflächen 2a, 3a, die an jeweiligen Halbschalen 2, 3 des Griffs 4 gebildet oder befestigt sind. Der Rand 18 jeder Großfläche 2, 3 und jeder Halbschale 2, 3 und damit die Form des gesamten Griffs 4 ist im weitesten Sinne trapezförmig mit leicht gekrümmten Rändern 18 und mit abgerundeten Ecken. Dadurch lässt sich der Griff 4 in einen im Querschnitt größeren ersten Griffabschnitt 4a und einen im Querschnitt kleineren zweiten Griffabschnitt 4b unterteilen. Der erste Griffabschnitt 4a ist zur Anlage von Daumen und Zeigefinger oder Mittelfinger des Patienten ausgebildet. Im zweite Griffabschnitt 4b ist ein Gewicht bzw. eine Masse 20 angeordnet, sodass dieser vom Daumen und Zeigefinger entfernte Griffabschnitt 4b stets in die Hand des Patienten fällt und/oder schwenkt.

### Bezugszeichenliste:

- 1: Handgerät
- 2: erste Halbschale
- 2a: erste Großfläche
- 3: zweite Halbschale
- 3a: zweite Großfläche
- 4: Griff
- 4a: erster Griffabschnitt
- 4b: zweiter Griffabschnitt
- 6: Signalleitung
- 8: Aktivierungsschalter
- 10: Findelicht
- 12: Etikett
- 14: Längsachse
- 16: Fingeraufnahme / Vertiefung
- 17: Stelle mit minimaler Größe
- 18: Rand
- 19: Volumenschwerpunkt
- 20: Gewichtskörper
- 21: Steckkontakt
- 22: Öffnung
- 23: Massenschwerpunkt
- 24: Entlüftungsöffnung
- 26: Strang

## Patentansprüche

1. Handgerät (1) zur patientenkontrollierten Analgesie mit einem Griff (4), welcher vorgesehen und ausgebildet ist, von einem Patienten mit einer Hand gegriffen und gehalten zu werden, und welcher ein Volumen mit einem Volumenschwerpunkt (19) einschließt oder definiert, wobei der Griff (4) einen ersten Griffabschnitt (4a) aufweist, welcher vorgesehen und ausgebildet ist, von einem Daumen und einem Zeigefinger oder einem Mittelfinger des Patienten derart gegriffen zu werden, dass ein an einer ersten Großfläche (2a) des Griffs (4) vorgesehener Aktivierungsschalter (8) von dem Daumen des Patienten betätigbar ist, während der Zeigefinger oder der Mittelfinger des Patienten in Anlage mit einer an einer zweiten Großfläche (3a) des Griffs (4) vorgesehenen definierten Fingeraufnahme (16) bringbar ist, und wobei der Griff (4) einen Massenschwerpunkt (23) aufweist, welcher bezüglich des Volumenschwerpunkts (19) des Griffs (4) in einer Richtung weg von dem ersten Griffabschnitt (4a) verschoben ist und sich in einem zweiten Griffabschnitt (4b) des Griffs (4) befindet.

2. Handgerät (1) nach Anspruch 1, **dadurch gekennzeichnet, dass** in dem zweiten Griffabschnitt (4b) ein Gewichtskörper (20) angeordnet ist.

3. Handgerät (1) nach Anspruch 2, **dadurch gekennzeichnet, dass** der Gewichtskörper (20) von einer Vergussmasse gebildet ist, die durch eine im Griff (4) angeordnete Öffnung (22) in den zweiten Griffabschnitt (4b) eingespritzt oder eingegossen ist.

4. Handgerät (1) nach Anspruch 3 wobei an dem Griff (4) eine Signalleitung (6) befestigt und angeschlossen ist, und wobei die Signalleitung (6) mit mindestens einem Strang (26) aus Para-Aramid verstärkt ist, der im Innern des Griffs (4) an diesem befestigt ist.

5. Handgerät (1) nach einem der vorhergehenden Ansprüche, wobei jede der beiden Großflächen (2a, 3a) zwei Ränder hat, die sich jeweils vom ersten Griffabschnitt (4a) zum zweiten Griffabschnitt (4b) erstrecken, wobei die beiden Ränder jeder Großfläche (2a, 3a) im ersten Griffabschnitt (4a) einen größeren Abstand zueinander haben, als im zweiten Griffabschnitt (4b).

6. Handgerät (1) nach Anspruch 5, **dadurch gekennzeichnet, dass** die Fingeraufnahme (16) von einer in der zweiten Großfläche (3a) rinnenförmigen oder trogförmigen Vertiefung gebildet ist, die sich durchgehend zwischen den beiden Rändern (18) der zweiten Großfläche (3a) erstreckt.

7. Handgerät (1) nach Anspruch 6, **dadurch gekennzeichnet, dass** die Vertiefung (16) ausgehend von einer Stelle (17) mit minimaler Größe in Richtung zu den beiden Rändern (18) jeweils zunehmende Größe aufweist.

8. Handgerät (1) nach Anspruch 7, **dadurch gekennzeichnet, dass** die Stelle (17) mit minimaler Größe mittig zwischen den beiden Rändern (18) der zweiten Großfläche (3a) angeordnet ist.

9. Handgerät (1) nach Anspruch 7 oder 8, **dadurch gekennzeichnet, dass** die Stelle (17) mit minimaler Größe gegenüber dem Aktivierungsschalter (8) angeordnet ist.

10. Handgerät (1) nach einem der vorhergehenden Ansprüche, **dadurch gekennzeichnet, dass** der Aktivierungsschalter (8) ein Folienschalter ist.

11. Handgerät (1) nach einem der vorhergehenden Ansprüche, **dadurch gekennzeichnet, dass** der Aktivierungsschalter (8) gegenüber der ersten Großfläche (2a) erhaben ist.

12. Handgerät (1) nach einem der vorhergehenden Ansprüche, **dadurch gekennzeichnet, dass** der Aktivierungsschalter (8) eine Signalfarbe aufweist, die von einer Farbe der ersten Großfläche (2a) abweicht.

13. Handgerät (1) nach einem der vorhergehenden Ansprüche, **dadurch gekennzeichnet, dass** an der ersten Großfläche (2a) oder an dem Aktivierungsschalter (8) eine als Findelicht (10) ausgebildete Beleuchtung vorgesehen ist.

14. Handgerät (1) nach einem der vorhergehenden Ansprüche, **dadurch gekennzeichnet, dass** an der ersten Großfläche (2a) oder an dem Aktivierungsschalter (8) eine als Freigabelicht ausgebildete Beleuchtung vorgesehen ist.

15. Handgerät (1) nach einem der vorhergehenden Ansprüche, **dadurch gekennzeichnet, dass** der Griff (4) eine Länge von 90 bis 110 mm hat.
